# EUROPEAN PATENT SPECIFICATION

(11) **EP 3 448 372 B1**
(45) Date of publication and mention of the grant of the patent: **20.10.2021**
(21) Application number: 17726694.7
(22) Date of filing: 20.04.2017
(51) Int. Cl.: A61K 31/12, A61K 31/575, A61K 31/353, A61P 13/00

(54) **FORMULATIONS FOR USE IN THE TREATMENT OR IN THE PREVENTION OF UROLOGICAL DISEASES**
FORMULIERUNGEN ZUR VERWENDUNG BEI DER BEHANDLUNG UND PRÄVENTION VON UROLOGISCHEN ERKRANKUNGEN
FORMULATIONS DESTINÉES À UNE UTILISATION DANS LE TRAITEMENT OU LA PRÉVENTION DE MALADIES UROLOGIQUES

(30) Priority: 29.04.2016 IT UA20163037
(43) Date of publication of application: 06.03.2019
(73) Proprietor: Inpha Research S.r.l., 20122 Milano MI (IT)
(72) Inventor: CASTELLI, Simone, 23900 Lecco (IT); SAMARITANI, Giuseppe, 23900 Lecco (IT); COSENTINO, Vincenzo, 23900 Lecco (IT)
(74) Representative: Perani & Partners S.p.A.
(86) International application number: PCT/IB2017/052268
(87) International publication number: WO 2017/187302

(56) References cited:
- WO-A1-2014/165971
- WO-A2-2006/087759
- US-A1- 2008 044 496
- US-A1- 2008 248 129
- SU KANG KIM ET AL: "Inhibitory effect of curcumin on testosterone induced benign prostatic hyperplasia rat model", BMC COMPLEMENTARY AND ALTERNATIVE MEDICINE, vol. 39, no. 1, 22 October 2015 (2015-10-22), page 69, XP055328092, DOI: 10.1002/biof.1066

## Description

### Field of the invention

The present invention relates to a combination of β-sitosterol, oligomeric proanthocyanidins, curcumin for use in the treatment of urological disorders and related formulations containing the aforesaid active principles in association with suitable excipients and/or diluents.

### State of the art

Benign prostatic hyperplasia (BPH) is the volumetric increase of the prostate gland (hyperplasia) located below the bladder. The prostate is a glandular structure whose function is producing the seminal fluid, the liquid conveying and feeding spermatozoa. Benign prostatic hyperplasia appears with a high frequency in males aged over 50, having incidence peaks higher than 50% in the age range comprised between 60 and 80 years. The main causes relate to a physiologic alteration of the hormonal axis due to the progressing age of the subject (reduction of the synthesis of androgenic hormones, andropause): in fact, the new hormone-secreting condition could favour an excessive stimulation of the prostate tissue, thus causing its hyperplasia (hyperproliferation). In any case, this hypothesis has not yet been definitely proved by sufficiently clear scientific data. Benign prostatic hyperplasia is a pathology that can be asymptomatic, even for a very long period. Only a significant volume increase of the prostate can cause the typical symptoms, such as difficult urination, prostate heaviness sensation, frequent and incomplete urination and even burning pain. These symptoms are attributable to a more or less important compression of the prostate on the urethra and to a consequent difficult urine outflow, which over time can lead to a weakening of the bladder tissue and to the formation of infections (urethritis) and of stones due to urinary stasis or bladder diverticula. The natural consequence is a weakening of the bladder and its consequent lower functional efficiency. The inadequate bladder emptying and the urine stasis facilitate the onset of infections or even the formation of stones. The pain, often associated with urinary stasis, infectious prostatitis and chronic damage to prostate-adjacent tissues (bladder) is supported by infectious and inflammatory phenomena backed by the liberation of prostaglandins, free radicals and other prostanoids and algogenic substances. These phenomena are often a relevant factor lowering the patient's quality of life and being hardly pharmacologically manageable with NSAIDs or other anti-inflammatory drugs due to the high average age of patients, to concomitant pharmacological therapies and to an impaired hepatorenal function. The diagnosis of prostatic hypertrophy is initially carried out by rectal exploration, the objective examination being performed by inserting a finger in the anal orifice. This examination is recommended every year starting from 50 years of age. As regards the instrumental diagnosis, the trans-rectal echography is the most widespread and less invasive test that allows viewing the actual size of the prostate and checking whether it presses on the urethra and the bladder. Urofluxometry is another routine investigation, which is scarcely invasive and can give an immediate feedback on the rate and strength of the urinary jet. The serological and urinary examination of the PSA, Prostate Specific Antigen, is carried out on subjects showing symptoms of a prostatic hyperplasia to exclude the presence of a prostate carcinoma.

Pharmacological therapies have the aim of reducing the volume of the prostate gland, lowering the pain associated with the inflammation of the prostate and of the adjacent tissues and improving the urinary flow and dynamics. The two major classes of drugs used in the IPB are the inhibitors of 5-α-reductase, such as dutasteride and finasteride, which inhibit the conversion of testosterone into dihydrotestosterone (DHT) that is mainly responsible for an enlarged prostate, and the α-lithics, such as silodosin, tamsulosin, terazosin and alfuzosin, which act primarily on the symptoms, thus favouring the relaxation of the bladder and prostate muscles and consequently improving the urinary flow. The main adverse events of the first group of drugs are, even if with low incidence, impotence and loss of libido, while in the second group the main adverse event is orthostatic hypotension (they are antihypertensive drugs). The surgical option is taken into account only in the event of a failure of the pharmacological therapy and consists in an endoscopic resection of the prostate (TURP). This surgical operation involves the removal of the prostatic adenoma by trans-urethral route, subsequently creating an anatomical space that facilitates urination. This is an only slightly invasive intervention that involves a hospital stay of a few days and a few weeks of convalescence.

Eating and life habits also play a key role. In particular, a prevailingly vegetarian diet plays a protective role, especially if it comprises vegetables with a high lycopene content like tomatoes, whose protective activity on the prostate tissue has now been ascertained. Even a poor intake of red meat and spirits and a regular physical activity play a clear protective role on the prostate tissue.

In recent years, a relevant role has been played by a phytotherapic approach to benign prostatic hyperplasia, which, under appropriate conditions, can significantly normalize the urinary flow and physiology and reduce the pain associated with the inflammation of the prostate and of the involved tissues. In particular, substances like phytosterols, with a specific reference to β-sitosterol contained in some plants such as *Serenoa repens* and in other plants such as the maritime pine, represent a milestone in the therapy against mild and moderate forms, exerting an inhibiting effect on the 5-α-reductase and on the inflammatory component. The same applies for polyphenol components, like oligomeric proanthocyanidins, extracted from plants such as pine and red vine, reducing the oxidative stress associated with prostate and bladder tissue damage, and curcumin, a substance having well-documented anti-inflammatory and ROS scavenging properties and, being already widely described in the biomedical bibliography for treating chronic inflammatory diseases such as Crohn's disease, inflammatory arthropathies and eye inflammations. Unfortunately, beside their well-documented pharmacological action, all these substances show a low oral bioavailability in humans and therefore often require high dosages for a minimal clinical efficacy. These dosages are scarcely compatible with an acceptably practical intake and have side effects.

US 2008/248129A1 discloses the treatment of benign prostatic hyperplasia (BPH) with herbal composition comprising β-sitosterol and oligomeric proanthocyanidins. Su Kang et al. 2015 discloses the effect of curcumin in reducing BPH.

WO 2014/165971A1 discloses compositions comprising curcumin and β-sitosterol for the treatment of BPH.

### Summary of the invention

The present invention relates to a combination of substances with anti-inflammatory activity, modulating the conversion of testosterone into dihydrotestosterone and antioxidant of vegetable origin, which can significantly reduce the inflammation and the hyperproliferation of the prostate tissue and of the adjacent tissues. The object of the present invention is therefore the combination of β-sitosterol, oligomeric proanthocyanidins and curcumin for use in the treatment and prevention of benign prostatic hyperplasia, wherein said combination is administered as an oral formulation in association with suitable excipients and/or diluents, wherein said excipients simultaneously contain bromelain, a sucroester, L-arginine base and piperine as enteric absorption promoter.

In particular, when the above oral formulations contain all four the aforesaid promoters, they show a significant anti-inflammatory effectiveness and reduce the prostatic volume at dosages of single active ingredients that are much lower than those described in the literature. This effectiveness is due to a mechanism for the promotion of enteric absorption not previously described, original and not explainable as the sum of the actions of the single promoters.

### Detailed description of the invention

For the purposes of the present invention, oligomeric proanthocyanidins are those polyphenols having a molecular weight comprised between 400 and 1000 Da, more preferably between 500 and 700 D.

In particular, the β-sitosterol contained in the combination for use according to the present invention is preferably contained in a plant extract with a titre not lower than 45%. This type of extracts are obtained, for example, from the pine bark (*Pinus pinaster Ait).*

For the purposes of the present invention, oligomeric proanthocyanidins are polyphenols with an average molecular weight comprised between 400 and 1000 Da, more preferably between 500 and 700 D.

The vegetable proanthocyanidins within the combination for use according to the present invention are preferably present in plant extracts with a titre not lower than 90% in oligomeric proanthocyanidins, for example obtained from the maritime pine and from the skins of *Vitis Vinifera.*

The curcumin in the combination for use according to the present invention is, for example, extracted from *Curcuma Longa.*

These synergistic active principles exert a significant anti-inflammatory, 5-α-reductase-inhibiting and antioxidant effect.

This combination of substances has proved particularly effective in reducing the inflammatory-congestive and hyperproliferating component of the prostate and urethral tissues (benign prostatic hyperplasia, prostatitis and urethritis).

This combination, although effective, especially if administered by oral route, requires rather high dosages to have clinically relevant effects, dosages that sometimes do not allow obtaining the necessary compliance of the patient and that sometimes might have unpleasant side effects, especially on patients who are sensitive to one or more of the active ingredients present in the combination.

As reported above, the Applicant has found that it is possible to overcome the above drawbacks with an oral formulation containing all four absorption promoters.

According to a further preferred embodiment, the oral formulations, beside containing the aforementioned promoters of enteric absorption, are also gastro-resistant and in particular are in the form of a tablet coated with gastro-resistant polymeric film or in the form of a hard capsule or a soft capsule (soft gel) whose shell is formed by conventional gastro-resistant polymers. This type of formulations is then capable of releasing said active principles in the small intestine in a highly bioavailable form due to the presence of all four promoters.

In particular, the promoters of enteric absorption are contained in each of said oral formulations according to the present invention in the following amounts:
- bromelain titre > 2000 GDU, from a minimum of 10 to a maximum of 50 mg for each administered dose;
- piperine from black pepper, from a minimum of 0.1 to a maximum of 5.0 mg for each administered dose;
- L-arginine base, from a minimum of 5 to a maximum of 50 mg for each administered dose;
- at least one sucroester, where sucroester means at least a sucrose ester with fatty acids, belonging to the category of food excipients indicated by the sygle E473, from a minimum of 5 to a maximum of 50 mg for each administered dose.

The aforesaid absorption promoters, in association between them, have surprisingly shown beneficial anti-inflammatory and anti-proliferative effects on patients affected by the aforesaid clinical conditions (with a specific reference to the IPB) thus reducing pain, burning sensation, difficult bladder emptying and foreign body sensation measured with algometric and self-assessment scales and through instrumental tests (urofluxometry and prostate scan), at dosages of the active principles that are distinctly lower than those cited in the literature in the absence of the aforesaid promoters. In particular, the combination of bromelain, piperine, L-arginine base and at least one sucroester, at dosages reported in the following formulation example, allows reducing the amount of curcumin from 1000 to 100 mg, the amount of oligomeric proanthocyanidins from 200 to 20 mg and the amount of β-sitosterol from 300 to 100 mg. The permanent effectiveness in spite of a significant reduction of the dosages of the active principles can be explained by the absorption-promoting effect exerted by the combination of bromelain, black pepper, at least one sucroester, L-arginine base, which produce a synergistic modulatory effect on the main anatomical-physiological mechanisms involved, thus hindering the enteric absorption of the active principles. In particular, bromelain can enhance the oral bioavailability of low molecular weight heparins, thus increasing their penetration through the intestinal epithelium (1). Probably, this effect is due to its proteolytic activity on the proteins composing the enteric mucus (mucins) (2). L-arginine favours the circulation of high molecular weight molecules by modifying the structure of the actin filaments at the tight junctions between enterocytes (zonula occludens) (3, 4).

Sucresters modulate the activity of some trans-membrane conveyors and alter the permeability of the cell membrane (5).

Piperine modulates the activity of hepatic and intestinal cytochromes, thus reducing the formation of less active metabolites and favouring their circulation (this mechanism is particularly important in the case of curcumin (6).

The increased bioavailability of the active principles mentioned in association with the aforesaid promoters of enteric absorption should neither be considered obvious or easily inferable by a person skilled in the art nor a mere additive sum of their actions, since such promoters act on very different mechanisms, and the scientific literature has ascertained their respective effectiveness on substrates that are substantially different from those object of the present invention. Therefore, a synergistic action in favour of an increased bioavailability must be reasonably traced back to synergistic mechanisms that cannot be easily described or hypothesized yet.

For instance, L-arginine, which is described in the literature as capable of transiently altering the integrity of structures of the cellular cytoskeleton like the actin filaments of the zonula occludens of the enterocyte, could also create the alkaline pH conditions for making polyphenolic substances such as curcumin and oligomeric proanthocyanidins, notoriously insoluble or poorly soluble in water, much more soluble in the enteric fluids and therefore more diffusible via the paracellular route. Moreover, the sucroester, known as a promoter of enteric absorption for increasing the diffusion capacity of the enterocyte cell membrane, could also create the conditions for dispersing β-sitosterol in micellar structures, making it more absorbable inside the enterocyte.

Therefore, the overall final effect of said promoters, attributable to the final effectiveness of the aforesaid composition, must be considered unexpected, not previously described and original.

This unexpected and surprising increased effectiveness of the formulation on inflammatory-congestive situations of the uro-genital tract and on prostatic hypertrophy allows treating patients who cannot take non-steroidal or steroidal anti-inflammatory drugs (due to renal, hypertensive or hepatic complications, gastric intolerance and osteoporosis) or α-lithic drugs and inhibitors of the 5-α-reductase like finasteride, with an effective preparation, having a good tolerability and being substantially free of significant adverse effects, so that it is suitable also for patients who have just undergone surgical treatments in the uro-genital area or are in treatment with different anti-platelet drugs, ACE-inhibitors and antihypertensive drugs.

The oral formulations according to the present invention can be used as food supplements or as proper pharmaceutical formulations.

According to a particularly preferred embodiment, the formulations suitable for oral administration according to the present invention contain:
- from 30 to 300 mg, and more preferably 100 mg, of β-sitosterol from various vegetable sources, e.g. from pine bark, with a minimum titre of 45% β-sitosterol;
- from 30 to 300 mg, and more preferably 100 mg, of curcumin, e.g. from *Curcuma longa* (minimum titre of 90% curcuminoids);
- from 10 to 100 mg, and more preferably 20 mg, of oligomeric proanthocyanidins extracted e.g. from the maritime pine bark *(Pinus pinaster Ait)* or from the red grape skin *(Vitis vinifera)* with a minimum titre of 90%;
   and the following absorption promoters in the following preferred amounts:
- from 10 to 50 mg, and more preferably 30 mg, of bromelain with a minimum titre of 2000 GDU;
- from 10 to 50 mg, and more preferably 20 mg, of L-arginine base,
- from 10 to 50 mg, and more preferably 30 mg, of a sucroester, meant as a sucrose ester with fatty acids belonging to the group of food excipients E473;
- from 0.1 to 5.0 mg, and more preferably 3.0 mg, of piperine from vegetable sources, e.g. from black pepper *(Piper Nigrum).*

The aforesaid pharmaceutical formulations or food supplements are preferably made in the form of tablets filmed with gastro-resistant polymers or in the form of a gastro-resistant rigid or soft (soft-gel) capsule, where gastro-resistance is obtained thanks to conventional polymers such as shellac, copolymeric derivatives of methacrylic acid (Eudragit) and other suitable components. The composition of an oral gastro-resistant formulation according to the present invention in the form of a shellac-filmed tablet is reported for illustrative and non-limitative purposes.

### Example 1

| | |
|---|---|
| Maritime pine bark extract | 135 mg |
| (Phytopin) Tit. 75% β-sitosterol | |
| Maritime pine bark extract | 21.0 mg |
| Tit. 95% OPC (as Pycnogenol) | |
| Curcuma longa root | 105 mg |
| Tit. 95% curcumin | |
| Bromelain 2500 GDU | 30.0 mg |
| L-arginine base | 20.0 mg |
| Piper Nigrum | 3.16 mg |
| Tit. 95 % piperine | |
| Sucroester (one or more belonging to E473) | 30.0 mg |
| Gastro-resistant excipients (shellac) | q.s. |
| Compression excipients | q.s. to 1250 mg |

### Bibliography

1. Grabovac V Bernkop-Schnürch A. Improvement of the intestinal membrane permeability of low molecular weight heparin by complexation with stem bromelain. Int J Pharm. 2006 Dec 1; 326(1-2): 153-9. Epub 2006 Jul 4;
2. Amini A et al. Depletion of mucin in mucin-producing human gastrointestinal carcinoma: Results from in vitro and in vivo studies with bromelain and N-acetylcysteine. Oncotarget. 2015 Oct 20; 6(32):33329-44;
3. Nusrat Abbas Motlekar, Kalkunte Srirangachar Srivenugopal, Mitchell S. Wachtel, and Bi-Botti Celestin Youan. Modulation of gastrointestinal permeability of low-molecular weight heparin by L-arginine: in-vivo and in-vitro evaluation. J Pharm Pharmacol. 2006 May; 58(5): 591-598;
4. Xue XY et al. Promoting effects of chemical permeation enhancers on insulin permeation across TR146 cell model of buccal epithelium in vitro. Drug Chem Toxicol. 2012 Apr; 35(2):199-207;
5. Kiss L et al. Sucrose esters increase drug penetration, but do not inhibit p-glycoprotein in caco-2 intestinal epithelial cells. J Pharm Sci. 2014 Oct; 103(10):3107-19;
6. Berginc K, Trontelj J, Basnet NS, Kristl A. Physiological barriers to the oral delivery of curcumin. Pharmazie. 2012 Jun; 67(6):518-24;

## Claims

1. Combination consisting of β-sitosterol, oligomeric proanthocyanidins and curcumin having anti-inflammatory and antiproliferative activity for use in the treatment and prevention of benign prostatic hyperplasia, wherein said combination is administered as an oral formulation in association with suitable excipients and/or diluents, wherein said excipients simultaneously contain bromelain, a sucroester, L-arginine base and piperine as enteric absorption promoter.

2. Combination for the use according to claim 1, in the form of a food supplement.

3. Combination for the use according to any one of claims 1-2, wherein:
a) β-sitosterol is present in said oral formulation at a dosage ranging from 30 to 300 mg and more preferably 100 mg per unitary dose and is in the form of plant extracts with a β-sitosterol titre not lower than 45%;
b) the oligomeric proanthocyanidins are present in said oral formulation at a dosage ranging from 10 to 100 mg and more preferably 20 mg per unitary dose and are in the form of plant extracts with an oligomeric proanthocyanidins titre not lower than 90%;
c) curcumin is present in said oral formulation at a dosage ranging from 30 to 300 mg and more preferably 100 mg per unitary dose in the form of plant extracts with a curcumin titre not lower than 90%.

4. Combination for the use according to any one of claims 1-3, wherein:
i) bromelain has a titre of at least 2000 GDU and is present in said oral formulation at a dosage ranging from 10 to 50 mg and more preferably 30 mg per unitary dose;
ii) L-arginine base is present in said oral formulation at a dosage ranging from 5 to 50 mg and more preferably 20 mg per unitary dose;
iii) at least one sucroester is present in said oral formulation at a dosage ranging from 5 to 50 mg and more preferably 30 mg per unitary dose;
iv) piperine is present in said oral formulation at a dosage from 0.1 to 5.0 mg and more preferably 3.0 mg per unitary dose in the form of plant extracts.

5. Combination for the use according to any one of claims 1-4, wherein said oral formulation is in the form of tablets coated with a gastro-resistant polymeric film or in the form of soft or rigid capsules, whose shell contains a gastro-resistant material.

## Patentansprüche

1. Kombination bestehend aus β-Sitosterol, oligomeren Proanthocyanidinen und Curcumin mit entzündungshemmender und antiproliferativer Wirkung zur Verwendung bei der Behandlung und Prävention von benigner Prostatahyperplasie, wobei die Kombination als orale Formulierung in Verbindung mit geeigneten Exzipienten und/oder Verdünnungsmitteln verabreicht wird, wobei die Exzipienten gleichzeitig Bromelain, einen Zuckerester, L-Arginin-Base und Piperin als enterischen Absorptionspromotor enthalten.

2. Kombination zur Verwendung nach Anspruch 1 in Form eines Nahrungsergänzungsmittels.

3. Kombination zur Verwendung nach einem der Ansprüche 1-2, wobei:
a) β-Sitosterin in der oralen Formulierung in einer Dosierung im Bereich von 30 bis 300 mg und bevorzugter 100 mg pro Einheitsdosis und in Form von Pflanzenextrakten mit einem β-Sitosterin-Titer von nicht weniger als 45 % vorhanden ist;
b) die oligomeren Proanthocyanidine in der oralen Formulierung in einer Dosierung im Bereich von 10 bis 100 mg und bevorzugter 20 mg pro Einheitsdosis und in Form von Pflanzenextrakten mit einem oligomeren Proanthocyanidin-Titer von nicht weniger als 90 % vorhanden sind;
c) Curcumin in der oralen Formulierung in einer Dosierung im Bereich von 30 bis 300 mg und bevorzugter 100 mg pro Einheitsdosis in Form von Pflanzenextrakten mit einem Curcumin-Titer von nicht weniger als 90 %

4. Kombination zur Verwendung nach einem der Ansprüche 1-3, wobei:
i) Bromelain einen Titer von mindestens 2000 GDU aufweist und in der oralen Formulierung in einer Dosierung im Bereich von 10 bis 50 mg und bevorzugter 30 mg pro Einheitsdosis vorhanden ist;
ii) L-Arginin-Base in der oralen Formulierung in einer Dosierung im Bereich von 5 bis 50 mg und bevorzugter 20 mg pro Einheitsdosis vorhanden ist;
iii) mindestens ein Zuckerester in der oralen Formulierung in einer Dosierung im Bereich von 5 bis 50 mg und bevorzugter 30 mg pro Einheitsdosis vorhanden ist;
iv) Piperin in der oralen Formulierung in einer Dosierung von 0,1 bis 5,0 mg und bevorzugter 3,0 mg pro Einheitsdosis in Form von Pflanzenextrakten vorhanden ist.

5. Kombination zur Verwendung nach einem der Ansprüche 1-4, wobei die orale Formulierung die Form von Tabletten, die mit einem magensaftresistenten Polymerfilm beschichtet sind, oder in Form von weichen oder starren Kapseln, deren Hülle ein magensaftresistentes Material enthält, aufweist.

## Revendications

1. Combinaison constituée de β-sitostérol, de proanthocyanidines oligomères et de curcumine ayant une activité anti-inflammatoire et antiproliférative pour une utilisation dans le traitement et la prévention de l'hyperplasie bénigne de la prostate, où ladite combinaison est administrée sous la forme d'une formulation orale en association avec des excipients et/ou diluants appropriés, où lesdits excipients contiennent simultanément de la bromélaïne, un sucroester, une base de L-arginine et de la pipérine en tant que promoteur d'absorption entérique.

2. Combinaison pour l'utilisation selon la revendication 1, sous la forme d'un complément alimentaire.

3. Combinaison pour l'utilisation selon l'une quelconque des revendications 1-2, où :
a) le β-sitostérol est présent dans ladite formulation orale à une dose comprise entre 30 et 300 mg et plus préférablement 100 mg par dose unitaire et se présente sous la forme d'extraits de plantes dont le titre en β-sitostérol n'est pas inférieur à 45% ;
b) les proanthocyanidines oligomères sont présentes dans ladite formulation orale à une dose comprise entre 10 et 100 mg et plus préférablement 20 mg par dose unitaire et se présentent sous la forme d'extraits de plantes dont le titre en proanthocyanidines oligomères n'est pas inférieur à 90% ;
c) la curcumine est présente dans ladite formulation orale à une dose comprise entre 30 et 300 mg et plus préférablement 100 mg par dose unitaire sous la forme d'extraits de plantes dont le titre en curcumine n'est pas inférieur à 90%.

4. Combinaison pour l'utilisation selon l'une quelconque des revendications 1-3, où :
i) la bromélaïne a un titre d'au moins 2000 GDU et est présente dans ladite formulation orale à une dose comprise entre 10 et 50 mg et plus préférablement 30 mg par dose unitaire ;
ii) la base de L-arginine est présente dans ladite formulation orale à une dose comprise entre 5 et 50 mg et plus préférablement 20 mg par dose unitaire ;
iii) au moins un sucroester est présent dans ladite formulation orale à une dose comprise entre 5 et 50 mg et plus préférablement 30 mg par dose unitaire ;
iv) la pipérine est présente dans ladite formulation orale à une dose de 0,1 à 5,0 mg et plus préférablement 3,0 mg par dose unitaire sous la forme d'extraits de plantes.

5. Combinaison pour l'utilisation selon l'une quelconque des revendications 1-4, où ladite formulation orale est sous la forme de comprimés enrobés d'un film polymère gastro-résistant ou sous la forme de capsules molles ou rigides, dont l'enveloppe contient un matériau gastro-résistant.
